(19) Europäisches Patentamt
European Patent Office
Office européen des brevets

(11) EP 2 373 670 B1

(12) # FASCICULE DE BREVET EUROPEEN

(45) Date de publication et mention de la délivrance du brevet:
**16.04.2014 Bulletin 2014/16**

(21) Numéro de dépôt: **09801479.8**

(22) Date de dépôt: **02.12.2009**

(51) Int Cl.:
***C07H 7/02*** (2006.01) ***A61K 8/60*** (2006.01)
***A61Q 19/08*** (2006.01) ***A61P 17/00*** (2006.01)
***C07D 309/10*** (2006.01) ***C07D 405/12*** (2006.01)
***C07D 233/56*** (2006.01) ***C07D 209/10*** (2006.01)
***A61K 31/351*** (2006.01) ***A61K 31/4178*** (2006.01)
***A61K 31/404*** (2006.01) ***A61K 8/49*** (2006.01)

(86) Numéro de dépôt international:
**PCT/FR2009/052378**

(87) Numéro de publication internationale:
**WO 2010/063953 (10.06.2010 Gazette 2010/23)**

(54) **NOUVEAUX COMPOSÉS C-XYLOSIDES AMINES ET UTILISATION EN COSMÉTIQUE**

NEUE AMINO SUBSTITUIERTE C-GLYKOSIDDERIVATE UND IHRE VERWENDUNG IN DER KOSMETIK

NOVEL AMINO SUBSTITUTED C-GLYCOSIDE DERIVATIVES AND USE THEREOF AS COSMETIC

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**
Etats d'extension désignés:
**AL BA RS**

(30) Priorité: **03.12.2008 FR 0858216**
**10.12.2008 US 121274 P**

(43) Date de publication de la demande:
**12.10.2011 Bulletin 2011/41**

(73) Titulaire: **L'Oréal**
**75008 Paris (FR)**

(72) Inventeurs:
- **DALKO, Maria**
**F-78000 Versailles (FR)**
- **CAVEZZA, Alexandre**
**F-93320 Pavillon Sous Bois (FR)**

(74) Mandataire: **Le Blainvaux Bellegarde, Françoise**
**L'Oréal**
**D.I.P.I.**
**25-29 Quai Aulagnier**
**92665 Asnières-sur-Seine Cedex (FR)**

(56) Documents cités:
**WO-A-02/051828 FR-A- 2 877 568**
**FR-A- 2 903 018**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 373 670 B1

## Description

La présente invention concerne de nouveaux dérivés C-xylosides aminés, les compositions notamment cosmétiques les comprenant, ainsi que leur utilisation pour lutter contre le vieillissement cutané.

Les femmes et les hommes ont tendance actuellement à vouloir paraître jeunes le plus longtemps possible et cherchent par conséquent à estomper les marques du vieillissement de la peau, qui se traduisent notamment par des rides et des ridules. A ce sujet, la publicité et la mode font état de produits destinés à garder le plus longtemps possible une peau éclatante et sans ride, marques d'une peau jeune, d'autant plus que l'aspect physique agit sur le psychisme et/ou sur le moral.

Jusqu'à présent, on traitait les rides et les ridules à l'aide de produits cosmétiques contenant des actifs agissant sur la peau, par exemple en améliorant son renouvellement cellulaire ou encore en favorisant la synthèse, ou en prévenant la dégradation, des fibres élastiques qui composent le tissu cutané.

On sait que la peau humaine est constituée de deux tissus l'un superficiel, l'épiderme, l'autre profond, le derme. L'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau, notamment le rôle de protection de l'organisme des agressions extérieures (climat, rayons ultraviolets, tabac, ...), appelé "fonction barrière".

Le derme fournit à l'épiderme un support solide. C'est également son élément nourricier. Il est principalement constitué de fibroblastes et d'une matrice extracellulaire composée majoritairement de collagène, d'élastine et d'une substance, dite substance fondamentale. Ces composants sont synthétisés par les fibroblastes. On y trouve aussi des leucocytes, des mastocytes ou encore des macrophages tissulaires. Enfin, le derme est traversé par des vaisseaux sanguins et des fibres nerveuses.

La matrice extracellulaire du derme est composée de protéines appartenant à plusieurs grandes familles : les collagènes, les glycoprotéines matricielles autres que les collagènes (fibronéctine, laminine), l'élastine et les protéoglycannes. On trouve également dans la matrice extracellulaire du derme des glycosaminoglycannes sous forme libre (c'est à dire non liés à une protéine).

Il est maintenant bien établi que des intéractions spécifiques existent entre ces différentes classes de protéines pour donner naissance à un tissu fonctionnel.

Un espace extracellulaire (micromatrice) existe également dans l'epiderme. Cet espace joue un rôle fonctionnel extrêmement important dans le renouvellement et ou le maintien du tissu cellulaire.

Les protéoglycannes sont des macromolécules complexes constituées d'un tronc protéique central ramifié, ou réseau de protéines, auquel sont attachés de très nombreuses chaînes latérales polyosidiques appelées glycosaminoglycannes.

Dans la suite de la présente demande, on désignera les protéoglycannes par l'abréviation PGs et les glycosaminoglycannes par l'abréviation GAGs.

Les GAGs ont longtemps été désignés sous le terme de mucopolysaccharides acides en raison de leur forte capacité de rétention d'eau, de leur nature glucidique et de leur caractère acide provenant de leurs multiples charges négatives.

Ainsi, la polarité des GAGs les fait implicitement participer à certaines fonctions biologiques comme l'hydratation des tissus, la fixation des cations ou le rôle de barrière de filtration ionique.

Les PGs et les GAGs sont synthétisés par différentes cellules au niveau du derme et de l'épiderme: fibroblastes, kératinocytes et mélanocytes.

Les fibroblastes synthétisent majoritairement des collagènes, des glycoprotéines matricielles autres que les collagènes (fibronéctine, laminine), des GAGs, des protéoglycannes et de l'élastine. Les kératinocytes synthétisent majoritairement des GAGs sulfatés et de l'acide hyaluronique alors que les mélanocytes ne produisent apparemment pas d'acide hyaluronique.

Lors de leur incorporation dans un PG, les GAGs sont sous la forme de chaînes linéaires composées de la répétition d'un diholoside de base contenant toujours une hexosamine (glucosamine ou galactosamine) et un autre ose (acide glucuronique, acide iduronique ou galactose). La glucosamine est soit N-sulfatée, soit N-acétylée. En revanche, la galactosamine est toujours N-acétylée. En outre, il peut y avoir des groupes sulfates O-liés sur l'hexosamine, l'acide uronique et le galactose.

Le fort caractère anionique des GAGs s'explique par la présence de groupes carboxylates au sein des acides hexuroniques (acide glucuronique et acide iduronique) et de groupes sulfate O- et N-liés.

Les principaux GAGs sont l'acide hyaluronique ou hyaluronanne (HA), l'héparane sulfate (HS), l'héparine (HP), la chondroïtine, la chondroïtine sulfate (CS), la chondroïtine 4-sulfate ou chondroïtine sulfate A (CSA), la chondroïtine 6-sulfate ou chondroïtine sulfate C (CSC), le dermatane sulfate ou chondroïtine sulfate B (CSB) et le kératane sulfate (KS) qui diffère des autres glycosaminoglycannes par la présence de galactose à la place de l'acide uronique.

Les protéoglycanes (PGs) sont formés par l'ancrage de plusieurs chaînes de GAGs sur une chaîne polypeptidique (dite protéine porteuse ou protéine « core »).

Les GAGs peuvent également exister dans la matrice extracellulaire sous forme libre c'est à dire non liée à une protéine matricielle : c'est notamment le cas de l'acide hyaluronique.

Lors de la synthèse des PGs, les GAGs sont polymérisés à partir de ces structures d'ancrage.

La synthèse des GAGs nécessite l'action coordonnée et concertée d'enzymes très spécifiques (transférases, épimérases, sulfotransférases) adjacentes dans la membrane du réticulum endoplasmique et de l'appareil de golgi. Puis une multitude de réactions biochimiques (N-désacétylation, N- et O-sulfatations, épimérisation) modifient les deux oses constitutifs du motif de base et cela de manière hétérogène le long de la chaîne. D'une chaîne d'héparane sulfate à l'autre par exemple, le rapport acide glucuronique/acide iduronique, la nature, le nombre et la position des O-sulfatations, ainsi que le rapport N-sulfate/O-sulfate peuvent varier, ce qui, potentiellement offre une immense diversité structurale.

De façon générale, les rôles biologiques des PGs sont très diversifiés, allant d'une fonction passive de support mécanique (par exemple serglycines) ou d'un rôle de barrière ionique de filtration moléculaire (par exemple perlecanne et bamacanne de la membrane basale glomérulaire), à des effets plus spécifiques dans l'adhésion, l'étalement, la prolifération, la différenciation cellulaire ou la morphogenèse, ou, à des effets très spécifiques d'interactions PG-protéine, tels que la fonction de récepteur du bétaglycanne ou l'interaction de la décorine avec le collagène. Ils jouent également un rôle fondamental dans la libération contrôlée de différents facteurs de croissance.

Un des rôles du tissu conjonctif dermique est de protéger l'organisme contre les agressions externes en formant en même temps une interface informative.

Pour ce faire, le derme possède une forte résistance mécanique en gardant, toutefois, une grande souplesse.

Sa résistance est assurée par le réseau dense des fibres de collagène, mais ce sont les PGs et l'acide hyaluronique, en assurant l'hydratation, la distribution et la souplesse des fibres qui font la différence entre la peau et, par exemple, le cuir.

Les PGs constituent 0,5 à 2 % du poids sec du derme, le collagène en représentant à lui seul jusqu'à 80 %.

La concentration et la distribution dans la peau humaine des GAGs et des PGs varient avec l'âge.

L'acide hyaluronique ou hyaluronanne (HA) est le principal GAG du derme, ce dernier renfermant la moitié d'HA de l'organisme.

La synthèse d'HA est effectuée notamment par les fibroblastes, à proximité de la face interne de la membrane plasmique. Elle s'effectue de façon continue. Ce polysaccharide gigantesque (plusieurs millions de daltons) possède une viscosité intrinsèque très élevée, assurant l'hydratation et l'assemblage des différents éléments du tissu conjonctif par formation de complexes supramoléculaires.

Le dermatanne sulfate (DS), initialement isolé du derme, est aussi très abondant dans la peau. Il constitue 40 à 50 % des GAGs dermiques.

Parallèlement aux mécanismes contribuant à l'élaboration de ces matrices extracellulaires spécialisées, il existe des processus de remodelage continuel dont la régulation dépend de la balance entre synthèse et dégradation des éléments protéiques de la matrice.

Plusieurs familles de protéases matricielles sont maintenant décrites ainsi que les facteurs intervenant dans leur activation-inactivation.

Au cours du vieillissement chronologique et/ou photoinduit, le derme et l'épiderme subissent de nombreuses modifications et dégradations qui se traduisent, avec l'âge, par une flaccidité et une perte de souplesse cutanée.

Parmi les éléments dégradés (notamment collagène et élastine), les PGs et les GAGs sont également altérés. En effet, au cours du vieillissement, les fibroblastes et les kératinocytes produisent de moins en moins de PGs et de GAGs et leur synthèse est imparfaite. Il en résulte une désorganisation importante : le dépôt des GAGs sur le squelette protéique formant le PG est anormal, ce qui a pour conséquence, une moins grande avidité pour l'eau de ces PGs et donc une diminution de l'hydratation et de la tonicité des tissus.

Restaurer une production normale de PGs et de GAGs par les fibroblastes et les kératinocytes contribue, en partie, à compenser la perte en hydratation cutanée.

La dégradation de ces matrices contribue donc au phénomène d'assèchement et de perte de souplesse de la peau.

On comprend donc l'importance de pouvoir disposer de produits dont les effets visent à maintenir le taux de PGs et de GAGs dans la peau et lui maintenir ainsi entre autre une bonne hydratation et une bonne souplesse.

Il est connu du document WO 02/051828 d'utiliser des composés C-glycosides pour augmenter la synthèse des glycosaminoglycannes par les fibroblastes et/ou les kératinocytes.

La demanderesse a découvert, de manière surprenante et inattendue, que d'autres dérivés C-glycosides aminés sont capables d'améliorer la synthèse des glycosaminoglycannes sulfatés comme la chondroïtine sulfate et le dermatane sulfate.

Leur efficacité est plus performante que celle des composés C-glycosides déjà connus. Ils sont plus efficaces pour améliorer le renouvellement épidermique et la fermeté de la peau et pour lutter plus efficacement contre les signes du vieillissement cutané. Ils ont également un effet bénéfique sur la structure de la jonction dermo-épidermique, notamment sur la cohésion entre derme et épiderme.

Ces nouveaux composés trouvent donc une application particulière dans les compositions cosmétiques ou dermatologiques, destinées à prévenir et/ou traiter cosmétiquement le vieillissement cutané; notamment prévenir et/ou traiter, en particulier par voie topique, les signes cutanés du vieillissement, et tout particulièrement les signes cutanés liés à une peau ridée, une peau présentant une altération de ses propriétés viscoélastiques ou biomécaniques, une peau présentant une altération dans la cohésion de ses tissus, une peau amincie et/ou une peau présentant une altération de son aspect de surface.

Les compositions selon l'invention peuvent permettre plus particulièrement de maintenir et/ou de restaurer les propriétés d'extensibilité, de tonicité, de fermeté, de souplesse, de densité et/ou d'élasticité de la peau.

Par "propriétés biomécaniques de la peau", on entend ici les propriétés d'extensibilité, de tonicité, de fermeté, de souplesse et/ou d'élasticité de la peau.

Par "signes cutanés du vieillissement", on entend ici toute modification de l'aspect extérieur de la peau due au vieillissement qu'il soit chronobiologique et/ou extrinsèque, en particulier photoinduit ou hormonal; parmi ces signes, on peut distinguer:

- la peau ridée, qui se traduit notamment par l'apparition de rides et/ou ridules,
- la peau présentant une altération de ses propriétés viscoélastiques ou biomécaniques, ou peau présentant un manque d'élasticité et/ou d'extensibilité et/ou de fermeté et/ou de souplesse et/ou de tonicité, qui se traduit notamment par une peau flétrie, molle, relâchée ou affaissée,
- la peau présentant une altération de la cohésion de ses tissus;
- la peau amincie,
- la peau présentant une altération de son aspect de surface, qui se traduit notamment par une altération du grain de la peau, par exemple une rugosité.

La présente invention a donc pour objet de nouveaux composés C-glycosides aminés de formule (I) telle que définie ci-après.

L'invention concerne également une composition cosmétique ou dermatologique comprenant, dans un milieu physiologiquement acceptable, au moins un tel composé.

Les composés selon l'invention répondent donc à la formule (I) suivante :

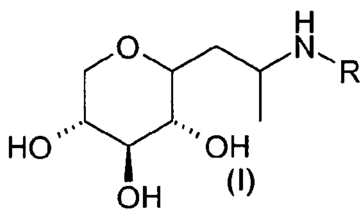

(I)

dans laquelle R représente :

- un radical alkyle linéaire en $C_2$-$C_{18}$, ou
- un radical alkyle cyclique en $C_3$-$C_{18}$, ou
- un radical alkyle ramifié en $C_3$-$C_{10}$, ou
- un radical -$CHR_1$-$COOR_2$ dans lequel :

$R_1$ représente H ou un radical alkyle linéaire en $C_1$-$C_6$, cyclique en $C_3$-$C_6$ ou ramifié en $C_3$-$C_6$, ledit radical pouvant être éventuellement (i) substitué par au moins un groupement choisi parmi =NH, -$NH_2$, -NHR', -$NR'_2$, =O, -OH, -OR', - SH, -SR', COOR', phényle, phényle substitué par OH ou OR',

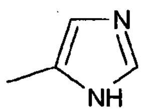 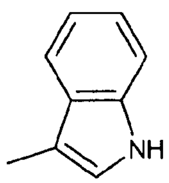 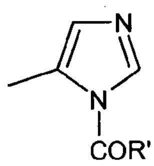 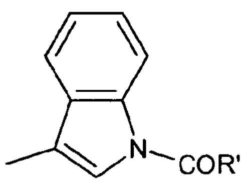

et/ou (ii) interrompu par un groupement -NH-, -N(COR')- ou -S-;

avec R' désignant un radical alkyle linéaire en $C_1$-$C_6$, cyclique en $C_3$-$C_6$, ou ramifié en $C_3$-$C_6$;

$R_2$ représente un radical alkyle linéaire en $C_1$-$C_6$, cyclique en $C_3$-$C_6$ ou ramifié en $C_3$-$C_6$ ; ainsi que leurs sels, leurs solvates et leurs isomères optiques.

Le terme alkyle, dans le cadre de la présente invention, signifie une chaîne hydrocarbonée saturée ou insaturée. Parmi les groupes alkyle convenant à la mise en ceuvre de l'invention, on peut notamment citer les groupes méthyle, éthyle, isopropyle, n-propyle, n-butyle, t-butyle, isobutyle, sec-butyle, pentyle, hexyle, heptyle, octyle, nonyle, décyle, cyclopropyle, cyclobutyle, cyclopentyle, cyclohexyle.

De préférence, R représente :

- un radical alkyle linéaire en $C_2$-$C_{16}$, ou
- un radical -$CHR_1$-$COOR_2$ dans lequel $R_1$ représente un radical alkyle linéaire en $C_1$-$C_4$ ou ramifié en $C_3$-$C_4$, éventuellement substitué par un groupement
- OH , COOR', phényle, phényle substitué par OH, R' représentant un radical alkyle linéaire en $C_1$-$C_4$, en particulier méthyle ou éthyle, ou un radical ramifié en $C_3$-$C_4$ ;

$R_2$ représente un radical alkyle linéaire en $C_1$-$C_4$, en particulier méthyle ou éthyle, ou un radical ramifié en $C_3$-$C_4$.

Préférentiellement, R représente :

- un radical alkyle linéaire en $C_2$-$C_{16}$, ou
- un radical -$CHR_1$-$COOR_2$ dans lequel $R_1$ un radical alkyle linéaire en $C_1$-$C_2$ ou ramifié en $C_3$-$C_4$, éventuellement substitué par un groupement -OH , un radical -benzyle, un radical hydroxy benzyle (notamment para hydroxy benzyle), un radical -$CH_2$-COOR' , R' représentant un radical alkyle linéaire en $C_1$-$C_4$, en particulier éthyle.

Dans un autre mode préféré de réalisation, lorsque R représente un radical -$CHR_1$-$COOR_2$ , $R_1$ représente le reste $R'_1$ d'un acide aminé de formule $NH_2$-$CHR'_1$-COOH, notamment d'un acide aminé choisi parmi :

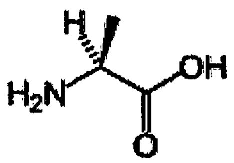
L-Alanine

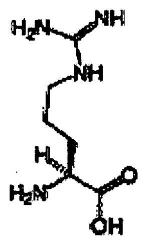
L-Arginine

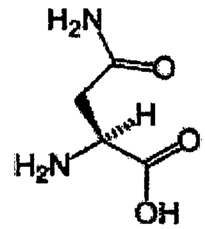
L-Asparagine

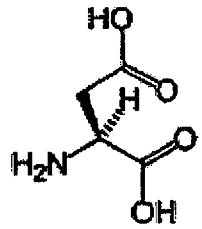
Acide L-aspartique

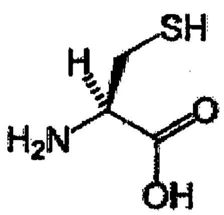
L-Cystéine

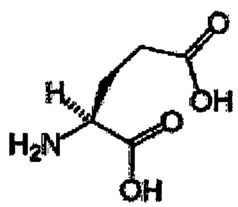
Acide L-glutamique

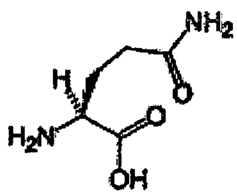
L-Glutamine

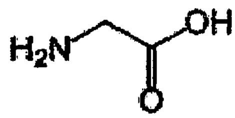
Glycine

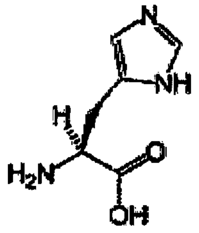
L-Histidine

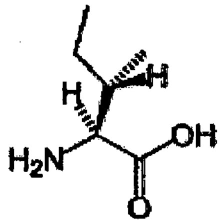
L-Isoleucine

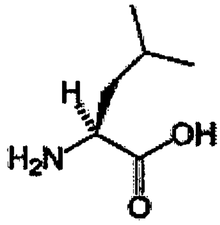
L-Leucine

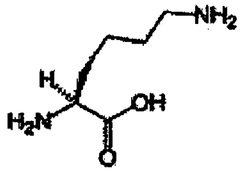
L-Lysine

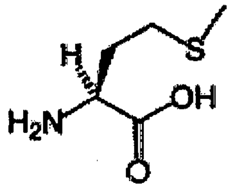
L-Méthionine

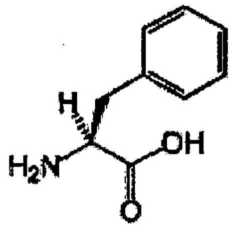
L-Phénylalanine

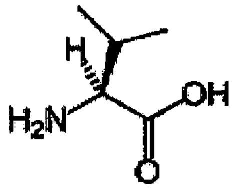
L-Valine

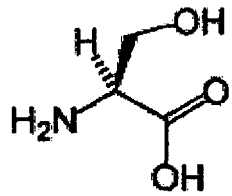
L-Sérine

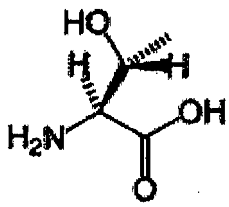
L-Thréonine

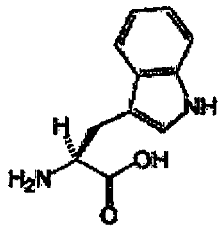
L-Tryptophane

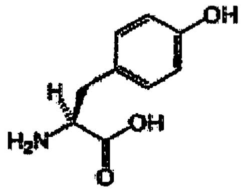
L-Tyrosine

Plus particulièrement, l'acide aminé peut être la phénylalanine (donc $R_1$ peut représenter $-CH_2$-phényle), la thréonine, la valine, l'acide aspartique, la tyrosine.

On utilise de préférence pour les composés de formule (I) ceux ayant les significations décrites ci-après :

Selon un premier mode de réalisation de l'invention, R désigne un radical alkyle linéaire en $C_2$-$C_{18}$.

Selon un second mode de réalisation de l'invention, R désigne un radical $-CHR_1-COOR_2$ dans lequel $R_1$ représente un radical alkyle linéaire en $C_1$-$C_4$ et $R_2$ représente un radical alkyle linéaire en $C_1$-$C_4$, en particulier éthyle.

Selon un troisième mode de réalisation de l'invention, R désigne un radical $-CHR_1-COOR_2$ dans lequel $R_1$ représente un radical alkyle ramifié en $C_3$-$C_4$ et $R_2$ représente un radical alkyle linéaire en $C_1$-$C_4$, en particulier éthyle.

Selon un quatrième mode de réalisation selon l'invention,
R désigne un radical $-CHR_1-COOR_2$ dans lequel $R_1$ représente un radical benzyle et $R_2$ représente un radical alkyle linéaire en $C_1$-$C_4$, en particulier éthyle.

Selon un cinquième mode de réalisation selon l'invention,
R désigne un radical $-CHR_1-COOR_2$ dans lequel $R_1$ représente un radical benzyle et $R_2$ représente un radical alkyle linéaire en $C_1$-$C_4$, en particulier éthyle.

On peut en particulier citer les composés suivants :

| Ex | R | R1 | R2 | NOM |
|---|---|---|---|---|
| **1** | $CHR_1-COOR_2$ | $-CH_2OH$ | $-C_2H_5$ | ester éthylique de l'acide 2R-[1-méthyl 2- (C- β-D-xylopyranoside) éthyl]-amino 3-hydroxy propanoïque, en particulier son chlorhydrate |
| **2** | $CHR_1-COOR_2$ | isopropyl | $-C_2H_5$ | ester éthylique de l'acide 2R-[1-méthyl 2- (C- β-D-xylopyranoside) éthyl]-amino 3-méthyl butanoïque, en particulier son chlorhydrate |
| **3** | $CHR_1-COOR_2$ | $-CH_2CO_2Et$ | $-C_2H_5$ | ester diéthylique du diacide 2R-[1-méthyl 2- (C- β-D-xylopyranoside) éthyl]-amino 1,4-butanoïque, en particulier son chlorhydrate |
| **4** | $CHR_1-COOR_2$ | -CH(OH)Me | $-CH_3$ | ester méthylique de l'acide 2R-[1-méthyl 2- (C- β-D-xylopyranoside) éthyl]-amino 3-hydroxy butanoïque, en particulier son chlorhydrate |
| **5** | $CHR_1-COOR_2$ | $-CH_2Ph$ | $-C_2H_5$ | ester éthylique de l'acide 2R-[1-méthyl 2- (C- β-D-xylopyranoside) éthyl]-amino 3-phényl propanoïque, en particulier son chlorhydrate |

(suite)

| Ex | R | R1 | R2 | NOM |
|---|---|---|---|---|
| **6** | $C_{12}H_{25}$ | - | - | 1-[2-(dodécylamino)-propyl]-C-β-D-xylopyranose) |
| **7** | $CHR_1$-$COOR_2$ | -$CH_2$(4-OH-Ph) | -$C_2H_5$ | ester éthylique de l'acide 2R-[1-méthyl 2- (C- β-D-xylopyranoside) éthyl]-amino 3-(4-hydroxy phényl) propanoïque, en particulier son chlorhydrate |
| **8** | $C_{16}H_{33}$ | - | - | 1-[2-(hexadécylamino)-propyl]-C-β-D-xylopyranose) |
| **9** | $C_8H_{17}$ | - | - | 1-[2-(octylamino)-propyl]-C-β-D-xylopyranose) |
| **10** | $C_6H_{13}$ | | | 1-[2-(hexylamino)-propyl]-C-β-D-xylopyranose) |
| **11** | $C_3H_7$ | | | 1-[2-(propylamino)-propyl]-C-β-D-xylopyranose) |

Les composés de formule (I) décrits précédemment peuvent également se présenter sous forme de sels, de solvates ou d'isomères optiques.

Comme sels des composés de formule (I), on peut citer les sels d'acides minéraux, tels que l'acide sulfurique, l'acide chlorhydrique, l'acide bromhydrique, l'acide iodhydrique, l'acide phosphorique, l'acide borique; ainsi que les sels d'acides organiques, tels que l'acide propionique, l'acide acétique, l'acide succinique, l'acide fumarique, l'acide lactique, l'acide glycolique, l'acide citrique et l'acide tartrique.

Les solvates acceptables des composés décrits dans la présente invention comprennent des solvates conventionnels tels que ceux formés lors de la dernière étape de préparation desdits composés du fait de la présence de solvants. A titre d'exemple, on peut citer les solvates dus à la présence d'eau ou d'alcools linéaires ou ramifiés comme l'éthanol ou l'isopropanol.

Les composés de formule (I) peuvent être préparés selon le schéma I ci-après :

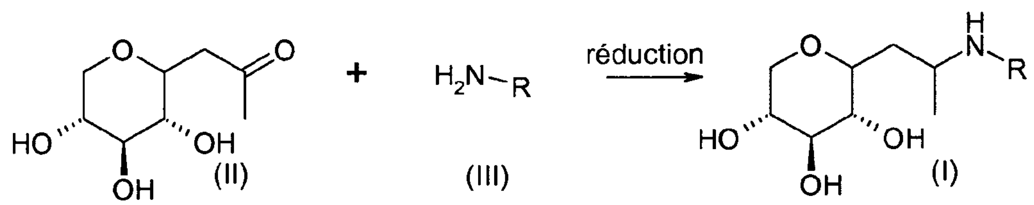


par réaction de la C-β-D-xylopyranoside-n-propane-2-one (II) (composé décrit à l'exemple 1 de la demande WO 02/051828) avec une amine R-$NH_2$ (III) dans laquelle R désigne un radical tel que défini précédemment dans les composés de formule (I) , notamment à une température comprise entre 15 et 60°C, de préférence à 25°C, dans un solvant polaire tel que l'éthanol; l'isopropanol, le méthanol, en présence de cyanoborohydrure de sodium de préférence en présence d'un acide organique (par exemple l'acide acétique) ou minéral (par exemple l'acide chlorhydrique), et d'un desséchant comme le sulfate de magnésium, le sulfate de sodium, ou un tamis moléculaire 4A, pendant 1 à 10 heures.

Le cyanoborohydrure de sodium peut être remplacé par du palladium sur charbon suivi d'une étape d'hydrogénation en présence d'hydrogène sous pression.

Le milieu réactionnel est filtré puis concentré sous pression réduite et purifié par exemple sur gel de silice, de manière à obtenir le produit recherché.

La présente invention concerne également une composition comprenant, dans un milieu physiologiquement acceptable, un composé de formule (I) tel que décrit ci-dessus. La composition est en particulier une composition cosmétique ou dermatologique.

Le composé de formule (I) peut être présent dans les compositions cosmétiques ou dermatologiques, en une quantité qui peut être comprise entre 0,01 et 10% en poids, de préférence entre 0,1 à 5% en poids, notamment entre 0,5 à 3% en poids, par rapport au poids total de la composition.

La composition comprend en outre un milieu physiologiquement acceptable, qui sera préférentiellement un milieu cosmétiquement ou pharmaceutiquement, notamment dermatologiquement, acceptable, c'est-à-dire sans odeur, couleur ou aspect désagréable, et qui ne génère pas de picotement, tiraillement ou rougeur inacceptable pour l'utilisateur. En particulier la composition est adaptée à une application topique sur la peau.

Par milieu physiologiquement acceptable, on comprend un milieu compatible avec les matières kératiniques d'êtres humains comme la peau du corps ou du visage, les lèvres, les muqueuses, les cils, les ongles, le cuir chevelu et/ou les cheveux.

La composition selon l'invention peut alors comprendre tous les adjuvants cosmétiques usuellement employés dans le domaine d'application envisagée.
On peut notamment citer l'eau; les solvants organiques, notamment les alcools en $C_1$-$C_6$ et les esters d'acide carboxylique en $C_2$-$C_{10}$; les huiles hydrocarbonées, les huiles siliconées, les huiles fluorées, les cires, les pigments, les charges, les colorants, les tensioactifs, les émulsionnants, les actifs cosmétiques ou dermatologiques, les filtres UV, les polymères filmogènes, les gélifiants hydrophiles ou lipophiles, les épaississants, les conservateurs, les parfums, les bactéricides, les absorbeurs d'odeur, les antioxydants.
Ces éventuels adjuvants peuvent être présents dans la composition à raison de 0,001 à 80% en poids, notamment 0,1 à 40% en poids, par rapport au poids total de la composition.
Ces adjuvants, selon leur nature, peuvent être introduits dans la phase grasse ou dans la phase aqueuse de la composition, ou dans des vésicules lipidiques. En tout état de cause, ces adjuvants, ainsi que leurs proportions, seront choisis par l'homme du métier de manière telle que les propriétés avantageuses des composés selon l'invention ne soient pas, ou substantiellement pas, altérées par l'adjonction envisagée.

Comme huiles utilisables dans l'invention, on peut citer les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matières grasses des alcools gras (alcool cétylique), des acides gras, des cires (cire de carnauba, ozokérite).

Comme gélifiants ou épaississants hydrophiles, on peut citer les polymères carboxyvinyliques (carbomer), les copolymères acryliques tels que les copolymères d'acrylates/alkylacrylates, les polyacrylamides, les polysaccharides, les gommes naturelles et les argiles; comme gélifiants ou épaississants lipophiles, on peut citer les argiles modifiées comme les bentones, les sels métalliques d'acides gras, et la silice hydrophobe.

Comme actifs, il sera avantageux d'introduire dans la composition utilisée selon l'invention au moins un composé choisi parmi: les agents desquamants; les agents hydratants; les agents dépigmentants ou propigmentants; les agents anti-glycation; les inhibiteurs de NO-synthase; les agents stimulant la synthèse de macromolécules dermiques ou épidermiques et/ou empêchant leur dégradation; les agents stimulant la prolifération des fibroblastes et/ou des kératinocytes ou stimulant la différenciation des kératinocytes; les agents myorelaxants et/ou les agents dermo-décontractants; les agents tenseurs; les agents anti-pollution et/ou anti-radicalaire; les agents agissant sur la microcirculation; les agents agissant sur le métabolisme énergétique des cellules; et leurs mélanges.
Des exemples de tels composés additionnels sont: le rétinol et ses dérivés tels que le palmitate de rétinyle; l'acide ascorbique et ses dérivés tels que l'ascorbyl phosphate de magnésium et le glucoside d'ascorbyle; le tocophérol et ses dérivés tels que l'acétate de tocophéryle; l'acide nicotinique et ses précurseurs tels que la nicotinamide; l'ubiquinone; le glutathion et ses précurseurs tels que l'acide L-2-oxothiazolidine-4-carboxylique; les extraits de plantes et notamment les protéines végétales et leurs hydrolysats, ainsi que les phytohormones; les extraits marins tels que les extraits d'algues; les extraits bactériens; les sapogénines telles que la diosgénine et les extraits de Wild Yam en contenant; les céramides; les hydroxyacides tels que l'acide salicylique et l'acide n-octanoyl-5-salicylique; le resvératrol; les oligopeptides et pseudodipeptides et leurs dérivés acylés; les sels de manganèse et de magnésium, en particulier les gluconates; et leurs mélanges.

Comme indiqué précédemment, la composition selon l'invention peut également renfermer des filtres UV ou agents photoprotecteurs actifs dans l'UVA et/ou l'UVB, sous forme de composés organiques ou inorganiques, ces derniers étant éventuellement enrobés pour les rendre hydrophobes.

Les agents photoprotecteurs organiques peuvent notamment être choisis parmi: les anthranilates, en particulier l'anthranilate de menthyle; les benzophénones, en particulier la benzophénone-1, la benzophénone-3, la benzophénone-5, la benzophénone-6, la benzophénone-8, la benzophénone-9, la benzophénone-12, et préférentiellement la Benzophénone-3 (Oxybenzone), ou la Benzophénone-4 (Uvinul MS40 de BASF); les benzylidènes-camphres, en particulier le 3-benzylidène-camphre, l'acide benzylidènecampho-sulfonique, le benzalkoniumméthosulfate de camphre, le polyacrylamidométhylbenzylidène camphre, l'acide téréphthalylidène di-camphre sulfonique, et préférentiellement le 4-méthylbenzylidène camphre (Eusolex 6300 de Merck); les benzimidazoles, en particulier le benzimidazilate (Neo Heliopan AP de Haarmann et Reimer), ou l'acide phénylbenzimidazole sulfonique (Eusolex 232 de Merck); les benzotriazoles, en particulier le drométrizole trisiloxane, ou le méthylène bisbenzotriazolyltétraméthylbutylphénol (Tinosorb M de Ciba); les cinnamates, en particulier le cinoxate, le DEA méthoxycinnamate, le méthylcinnamate de diisopropyle, le glycéryl éthylhexanoate de diméthoxycinnamate, le méthoxycinnamate d'isopropyle, le cinnamate d'isoamyle, et préférentiellement l'éthocrylène (Uvinul N35 de BASF), l'octylméthoxycinnamate (Parsol MCX de Hoffmann La Roche), ou l'octocrylène (Uvinul 539 de BASF); les dibenzoylméthanes, en particulier le butyl méthoxydibenzoylméthane (Parsol 1789); les imidazolines, en particulier l'éthylhexyl diméthoxybenzylidène dioxoimidazoline; les PABA, en particulier l'éthyl Dihydroxypropyl PABA, l'éthylhexyldiméthyl PABA, le glycéryl PABA, le PABA, le PEG-25 PABA, et préférentiellement la diéthylhexylbutamido-triazone (Uvasorb HEB de 3V Sigma), l'éthylhexyltriazone (Uvinul T150 de BASF), ou l'éthyl PABA (benzocaïne); les salicylates, en particulier le salicylate de dipropylèneglycol, le salicylate d'éthylhexyle, l'homosalate, ou le TEA salicylate; les triazines, en particulier l'anisotriazine (Tinosorb S de Ciba); le drometrizole trisiloxane.

Les agents photoprotecteurs inorganiques sont de préférence constitués d'oxyde de zinc et/ou de dioxyde de titane, de préférence de taille nanométrique, éventuellement enrobés d'alumine et/ou d'acide stéarique.

Cette composition peut se présenter sous toutes les formes galéniques normalement utilisées dans le domaine cosmétique ou dermatologique, et notamment sous forme d'une solution aqueuse ou hydroalcoolique, éventuellement gélifiée, d'une dispersion du type lotion éventuellement biphasée, d'une émulsion obtenue par dispersion d'une phase grasse dans une phase aqueuse (H/E) ou inversement (E/H), ou d'une émulsion triple (E/H/E ou H/E/H) ou d'une dispersion vésiculaire de type ionique et/ou non ionique; de gel aqueux ou huileux. Ces compositions sont préparées selon les méthodes usuelles. On préfère utiliser selon cette invention une composition sous la forme d'une émulsion notamment huile-dans-eau.

La composition peut être plus ou moins fluide et avoir l'aspect d'une crème blanche ou colorée, d'une pommade, d'un lait, d'une lotion, d'un sérum, d'une pâte, d'un gel, d'une mousse. Elle peut éventuellement être appliquée sous forme d'aérosol. Elle peut également se présenter sous forme solide, en particulier sous forme de stick.

Lorsque la composition est une émulsion, la proportion de la phase grasse peut aller de 5 à 80% en poids, de préférence de 8 à 50% en poids par rapport au poids total de la composition. L'émulsionnant et le co-émulsionnant peuvent être présents en une proportion allant de 0,3 à 30% en poids, et de préférence de 0,5 à 20% en poids, par rapport au poids total de la composition.

La composition selon l'invention peut constituer une composition de soin de la peau, et notamment une crème de nettoyage, de protection, de traitement ou de soin pour le visage, pour les mains, pour les pieds, pour les grands plis anatomiques ou pour le corps (par exemple crèmes de jour, crèmes de nuit, crèmes démaquillantes, crèmes de fond de teint, crèmes anti-solaires); un lait de démaquillage, un lait corporel de protection ou de soin, un lait anti-solaire; une lotion, gel ou mousse pour le soin de la peau, comme une lotion de nettoyage.

La composition selon l'invention est avantageusement une composition anti-âge, notamment de soin, destinée à traiter et/ou lutter contre, cosmétiquement, les signes extérieurs du vieillissement cutané; la composition est plus particulièrement une composition de soin des peaux matures.

La composition peut être également une composition de maquillage, notamment un fond de teint.

L'invention concerne également un procédé de traitement cosmétique de la peau, comprenant l'application sur la peau, d'une composition cosmétique telle que définie ci-dessus. Ce procédé trouve une application avantageuse dans le traitement de la peau, notamment de la peau mature et/ou de la peau ridée, en particulier du visage, notamment du front, du cou et/ou des mains.

L'invention concerne également l'utilisation d'une composition cosmétique telle que définie ci-dessus ou d'un composé de formule (I) tel que décrit précédemment, pour prévenir et/ou traiter cosmétiquement les signes cutanés du vieillissement, notamment les signes cutanés choisis parmi la peau ridée, la peau présentant une altération de ses propriétés viscoélastiques ou biomécaniques, la peau présentant une altération dans la cohésion de ses tissus, la peau amincie, et la peau présentant une altération de son aspect de surface.

L'invention concerne aussi l'utilisation cosmétique d'une composition telle que définie ci-dessus ou d'un composé de formule (I) tel que décrit précédemment, pour améliorer la fermeté de la peau et/ou pour améliorer la structure de la jonction dermo-épidermique et/ou pour renforcer la cohésion entre le derme et l'épiderme.

L'invention est illustrée plus en détail par les exemples non limitatifs suivants.

**<u>Exemple 1 : Synthèse du chlorhydrate de l'ester éthylique de l'acide 2R-[ 1-méthyl 2-(C-β-D-xylopyranoside)éthyl]-amino 3-hydroxy propanoïque</u>**

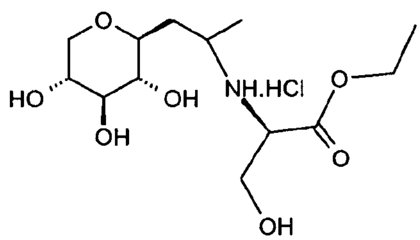

Ce composé comprend un groupe R qui est le reste d'un résidu sérine.

Dans 20 ml d'éthanol, on a fait réagir 2 g (11,8 mmol, 1 eq) du chlorhydrate de l'ester éthylique de la sérine avec 2,98 g (15,7 mmol, 1,33 eq) de C-β-D-xylopyranoside-n-propane-2-one, 0,99 g de $NaBH_3CN$ (15,7 mmol, 1,33 eq), 0.7 ml d'acide acétique (11,8 mmol, 1 eq) et 2 spatules de sulfate de sodium pendant 12 heures à température ambiante (25 °C).

A l'issue de la réaction, le milieu réactionnel a été filtré, concentré sous vide puis purifié sur gel de silice (dichlorométhane/méthanol)

On a obtenu 300 mg de produit (rendement 23%) sous forme d'une poudre blanche.
Les spectres RMN $^1$H et de masse sont conformes à la structure du produit attendu.

**Exemple 2 : Synthèse du chlorhydrate de l'ester éthylique de l'acide 2R-[ 1-méthyl 2- (C- β-D-xylopyranoside) éthyl]-amino 3-méthyl butanoïque**

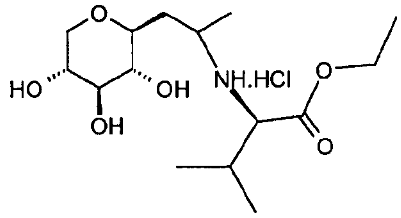

Ce composé comprend un groupe R qui est le reste d'un résidu valine.

Le composé a été préparé selon le procédé de synthèse décrit à l'exemple 1 en utilisant 2 g de chlorhydrate de l'ester éthylique de valine, 2,79 g de C-β-D-xylopyranoside-n-propane-2-one, 0,917 g de $NaBH_3CN$, 0,65 ml d'acide acétique, et 2 spatules de sulfate de sodium, dans 25 ml d'éthanol.

On a obtenu 800 mg (rendement 23 %) de produit sous forme d'une huile jaune claire.

Les spectres RMN $^1$H et de masse sont conformes à la structure du produit attendu.

**Exemple 3 : Synthèse du chlorhydrate de l'ester diéthylique du diacide 2R-[1-méthyl 2. (C- β-D-xylopyranoside) éthyl]-amino 1,4-butanoïque**

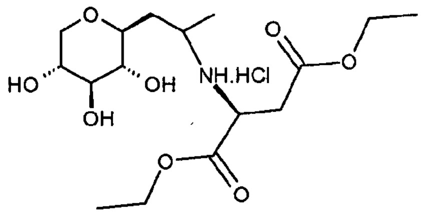

Ce composé comprend un groupe R qui est le reste d'un résidu acide aspartique.

Le composé a été préparé selon le procédé de synthèse décrit à l'exemple 1 en utilisant 2 g de chlorhydrate du diester éthylique d'acide aspartique, 2,24 g de C-β-D-xylopyranoside-n-propane-2-one, 0,741 g de $NaBH_3CN$, 0,53 ml d'acide acétique, et 2 spatules de sulfate de sodium, dans 20 ml d'éthanol.

On a obtenu 940 mg (rendement 29 %) de produit sous forme d'une huile incolore.

Les spectres RMN $^1$H et de masse sont conformes à la structure du produit attendu.

**Exemple 4 : Synthèse du chlorhydrate de l'ester méthylique de l'acide 2R-[1-méthyl 2- (C- β-D-xylopyranoside) éthyl]-amino 3-hydroxy butanoïque**

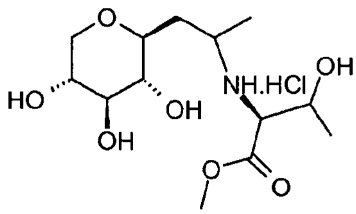

Ce composé comprend un groupe R qui est le reste d'un résidu thréonine.

Le composé a été préparé selon le procédé de synthèse décrit à l'exemple 1 en utilisant 0,5 g de chlorhydrate de l'ester méthylique de thréonine, 0,75 g de C-β-D-xylopyranoside-n-propane-2-one, 0,25 g de $NaBH_3CN$, 70 μl d'acide acétique, et 1 spatules de sulfate de sodium, dans 7 ml d'éthanol.

On a obtenu 199 mg (rendement 22 %) de produit sous forme d'une poudre blanche.

Les spectres RMN $^1H$ et de masse sont conformes à la structure du produit attendu.

**<u>Exemple 5 : Synthèse du chlorhydrate de l'ester éthylique de l'acide 2R-[ 1-méthyl 2- (C- β-D-xylopyranoside) éthyl]-amino 3-phényl propanoïque</u>**

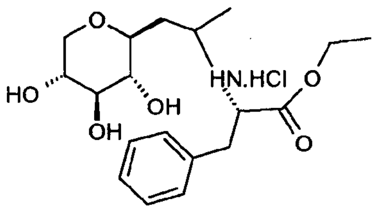

Ce composé comprend un groupe R qui est le reste d'un résidu phénylalanine.

Le composé a été préparé selon le procédé de synthèse décrit à l'exemple 1 en utilisant 3,5 g de chlorhydrate de l'ester éthylique de phénylalanine, 2,65 g de C-β-D-xylopyranoside-n-propane-2-one, 1,02 g de $NaBH_3CN$, 0,73 ml d'acide acétique, et 2 spatules de sulfate de sodium, dans 30 ml d'éthanol.

On a obtenu 3,9 g (rendement 69 %) de produit sous forme d'une poudre blanche.

Les spectres RMN $^1H$ et de masse sont conformes à la structure du produit attendu.

**<u>Exemple 6 : Synthèse du 1-[2-(dodécylamino)-propyl]-C-β-D-xylopyranose)</u>**

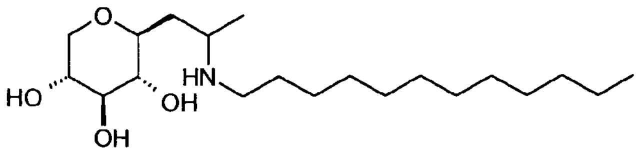

Le composé a été préparé selon le procédé de synthèse décrit à l'exemple 1 en utilisant 2,97 g de dodécylamine, 2,70 g de C-β-D-xylopyranoside-n-propane-2-one, 1,02 g de $NaBH_3CN$, 0,9 ml d'acide acétique, et 2 spatules de sulfate de sodium, dans 30 ml d'éthanol.

On a obtenu 2,3 g (rendement 41 %) de produit sous forme d'une cire blanche.

Les spectres RMN $^1H$ et de masse sont conformes à la structure du produit attendu.

**<u>Exemple 7 : Synthèse du chlorhydrate de l'ester éthylique de l'acide 2R-[ 1-méthyl 2- (C- β-D-xylopyranoside) éthyl]-amino 3-(4-hydroxy phényl) propanoïque</u>**

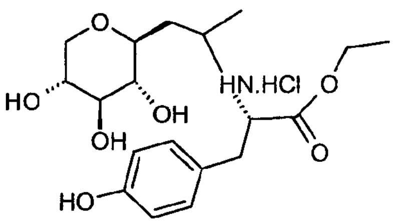

Ce composé comprend un groupe R qui est le reste d'un résidu tyrosine.

Le composé a été préparé selon le procédé de synthèse décrit à l'exemple 1 en utilisant 2 g de chlorhydrate de l'ester éthylique de tyrosine, 2,06 g de C-β-D-xylopyranoside-n-propane-2-one, 0,68 g de $NaBH_3CN$, 0,48 ml d'acide acétique, et 2 spatules de sulfate de sodium, dans 25 ml d'éthanol.

On a obtenu 800 mg (rendement 23 %) de produit sous forme d'une huile orange.

Les spectres RMN $^1H$ et de masse sont conformes à la structure du produit attendu.

__Exemple 8 : Synthèse du 1-[2-(hexadécylamino)-propyl]-C-β-D-xylopyranose)__

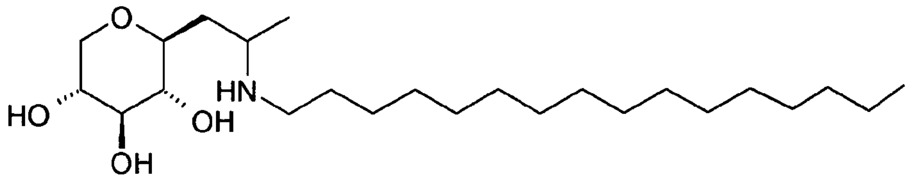

Le composé a été préparé selon le procédé de synthèse décrit à l'exemple 1 en utilisant 1,3 g d'hexadécylamine, 1 g de C-β-D-xylopyranoside-n-propane-2-one, 0,36 g de $NaBH_3CN$, 0,3 ml d'acide acétique, et 1 spatule de sulfate de sodium, dans 11 ml d'éthanol.

On a obtenu 1,77 g (rendement 87 %) de produit sous forme d'une gomme blanche.

Les spectres RMN $^1H$ et de masse sont conformes à la structure du produit attendu.

__Exemple 9 : Synthèse du 1-[2-(octylamino)-propyl]-C-β-D-xylopyranose)__

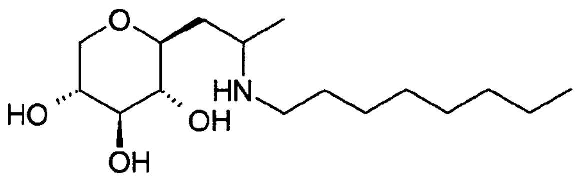

Le composé a été préparé selon le procédé de synthèse décrit à l'exemple 1 en utilisant 0,81 g d'octylamine, 1 g de C-β-D-xylopyranoside-n-propane-2-one, 0,36 g de $NaBH_3CN$, 0,3 ml d'acide acétique, et 1 spatule de sulfate de sodium, dans 11 ml d'éthanol.

On a obtenu 0,9 g (rendement 57 %) de produit sous forme d'une gomme blanche.

Les spectres RMN $^1H$ et de masse sont conformes à la structure du produit attendu.

__Exemple 10 : Synthèse du 1-[2-(hexylamino)-propyl]-C-β-D-xylopyranose)__

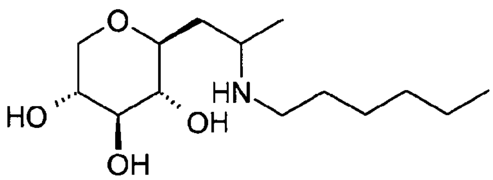

Dans 50 ml d'éthanol, on a fait réagir 1,2 g (12 mmol) d'hexylamine avec 1,9 g (10 mmol) de C-β-D-xylopyranoside-n-propane-2-one, 0,2 g de catalyseur au palladium supporté à 10 % sur du charbon (0,1 eq), 0,2 g d'acide acétique. Le mélange a été ensuite hydrogéné sous une pression d'hydrogène à 5,06 x $10^5$ Pa (5 atmosphères) pendant 40 heures.

A l'issue de la réaction, le milieu réactionnel a été filtré, concentré sous vide puis purifié sur gel de silice (dichlorométhane/méthanol)

On a obtenu 402 mg de produit (rendement 15 %) sous forme d'une huile jaune.

Les spectres RMN $^1H$ et de masse sont conformes à la structure du produit attendu.

**Exemple 11** : **Synthèse du 1-[2-(propylamino)-propyl]-C-β-D-xylopyranose)**

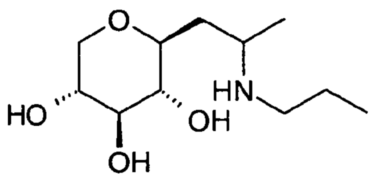

Le composé a été préparé selon le procédé de synthèse décrit à l'exemple 10 en utilisant 1,2 eq de propylamine, 1 eq de C-β-D-xylopyranoside-n-propane-2-one, 0,1 eq de catalyseur au palladium supporté à 10 % sur du charbon, 1,2 eq d'acide acétique, dans l'éthanol.

On a obtenu 500 mg (rendement 25 %) de produit attendu.

Les spectres RMN $^1H$ et de masse sont conformes à la structure du produit attendu.

**Exemple 12**

Etude de l'effet de dérivés C-glycosides sur la synthèse des glycosaminoglycannes sulfatés sur fibroblastes et sur kératinocytes :

L'étude est faite par mesure de l'incorporation de glucosamine radioactive dans la matrice néosynthétisée par des cultures de fibroblastes dermiques humains normaux ou par des kératinocytes. L'incorporation de glucosamine radioactive indique une néosynthèse spécifique de glycosaminoglycannes *via* une incorporation de la forme acétylée de cette glucosamine.

Les cultures de fibroblastes sont effectuées selon les méthodes classiques de culture cellulaire, à savoir en milieu DMEM vendu par la société Gibco, en présence de L-glutamine (2 mM), de pénicilline (50 UI/ml) et de 10% de sérum de veau foetal (Gibco).

Les cultures de kératinocytes sont effectuées en milieu Keratinocyte-SFM vendu par la société Gibco , en présence d'EGF (Epidermal Growth Factor) (0,25 ng/ml), d'extrait pituitaire (25 μg/ml) et de gentamycine (25 μg/ml).

Les fibroblastes et les kératinocytes ont été cultivés en plaques de 96 puits. A confluence, le milieu de culture a été remplacé par du milieu de culture adéquat contenant ou non (témoin) le composé à tester ou la référence, puis les cellules ont été incubées pendant 48 heures. Le marqueur radioactif 35 S-sulfate a été ajouté (40 μCi final) et les cellules ont été incubées pendant 24 heures supplémentaires. Toutes les conditions ont été réalisées 3 fois.

Les glycosaminoglycanes de la matrice extracellulaire ont été extraits avec un volume tampon chaotropique (Tris/HCl 50 mM, guanidine 4 M, EDTA 5 mM, pH 8,0).

Les GAGs sulfatés ont été purifiés par chromatographie d'échanges d'ions : absorption des molécules anioniques sur billes de Q-Sépahrose dans des conditions de forte stringence, désorption des molécules peu et moyennement anioniques avec uns solution urée 6 M NaCl 0,2 mM, puis lavages.

La radioactivité incorporée dans les molécules très cationiques restées sur le support (GAGs majoritairement) a été mesurée par scintillation liquide.

Les résultats sont évalués par apport à un témoin constitué par des cellules n'ayant pas été traitées avec un composé de formule (I).

Un témoin positif (TGFβ à 10 ng/ml) connu pour stimuler la synthèse des GAGs est introduit dans le test effectéu sur fibroblastes comme référence positive.

Un témoin positif ($CaCl_2$ à 1,4 mM) connu pour stimuler la synthèse des GAGs est introduit dans le test effectué sur kératinocytes comme référence positive.

Les résultats sont exprimés en pourcentage de variation de la synthèse des glycosaminoglycanes par rapport au témoin.

On a effectué 3 essais pour le composé testé.

Les comparaisons des résultats obtenus pour un composé testé ont été réalisés à l'aide du test de Student.

L'écart type de la moyenne (esm) a été calculé selon la relation suivante :

$$esm = \text{écart type} / (n)^{1/2}$$

n étant le nombre d'essais effectués.

Les résultats sont présentés dans le tableau suivant :

| **Traitement sur fibroblastes** | | Valeur moyenne | esm | n | % | p |
|---|---|---|---|---|---|---|
| Contrôle (milieu de culture) | | 2332 | 7 | 3-3 | 100-100 | 0,01 à 0,05 |
| Contrôle positif (TGFβ) | | 4062 | 9 | 3-3 | 174 | <0,001 |
| Composé de l'exemple 5 | 0,37 mM<br>1,11 mM | 14373<br>14111 | 24<br>22 | 3<br>3 | 616<br>605 | <0,001<br><0,001 |

| **Traitement sur kératinocytes** | | Valeur moyenne | esm | n | % | p |
|---|---|---|---|---|---|---|
| Contrôle (milieu de culture) | | 4526 | 4 | 3-3 | 100-100 | 0,01 à 0,05 |
| Contrôle positif ($CaCl_2$) | | 5273 | 3 | 3-3 | 117 | 0,001 à 0,01 |
| Composé de l'exemple 5 | 66 μM<br>200 μM | 21975<br>29007 | 14<br>2 | 3<br>3 | 438<br>570 | <0,001<br><0,001 |

Les valeurs mesurées sont données en coups par minute (cpm)

n : nombre d'essais effectués
p : intervalle de confiance
esm : écart type de la moyenne

Dans un autre plan d'expérience, on a évalué les composés des exemples 10 et 11 en comparaison avec 3 composés ne faisant pas partie de l'invention.

| **Traitement sur fibroblastes** | | Valeur moyenne | esm | n | % |
|---|---|---|---|---|---|
| Contrôle (milieu de culture) | | 2518 | 2 | 2 | 100 |
| Composé de l'exemple 10 | 0,1 mM<br>1 mM | 13925<br>14992 | 6<br>35 | 2<br>2 | 553<br>595 |
| Composé de l'exemple 11 | 0,1 mM<br>1 mM | 3197<br>11109 | 8<br>0 | 2<br>2 | 127<br>441 |
| 1-[2-amino-propyl]-C-β-D-xylopyranose (composé décrit dans WO 02/051828) | 0,1 mM<br>1 mM | 2301<br>3507 | 9<br>17 | 2<br>2 | 91<br>139 |
| 1-[2-(méthylamino)-propyl]-C-β-D-xylopyranose (composé hors invention) | 0,1 mM<br>1 mM | 2569<br>2659 | 6<br>6 | 2<br>2 | 102<br>106 |
| 1-[2-(3-hydroxy-propylamino)-propyl]-C-β-D-xylopyranose (composé de l'exemple 9 du WO 02/051828) | 0,1 mM<br>1 mM | 2856<br>3039 | 1<br>0 | 2<br>2 | 113<br>121 |

| **Traitement sur kératinocytes** | | Valeur moyenne | esm | n | % |
|---|---|---|---|---|---|
| Contrôle (milieu de culture) | | 4526 | 4 | 2 | 100 |
| Composé de l'exemple 10 | 0,1 mM<br>1 mM | 54897<br>32733 | 8<br>0 | 2<br>2 | 427<br>255 |

(suite)

| Traitement sur kératinocytes | | Valeur moyenne | esm | n | % |
|---|---|---|---|---|---|
| Composé de l'exemple 11 | 0,1 μM<br>1 μM | 14408<br>45124 | 7<br>6 | 2<br>2 | 112<br>351 |
| 1-[2-amino-propyl]-C-β-D-xylopyranose (composé décrit dans WO 02/051828) | 0,1 mM<br>1 mM | 14465<br>28870 | 6<br>11 | 2<br>2 | 113<br>225 |
| 1-[2-(méthylamino)-propyl]-C-β-D-xylopyranose (composé hors invention) | 0,1 mM<br>1 mM | 11922<br>17916 | 6<br>11 | 2<br>2 | 93<br>139 |
| 1-[2-(3-hydroxy-propylam ino)-propyl]-C-β-D-xylopyranose (composé de l'exemple 9 du WO 02/051828) | 0,1 mM<br>1 mM | 12660<br>18691 | 1<br>5 | 2<br>2 | 99<br>145 |

Les résultats obtenus montrent de façon significative que les composés 5, 10 et 11 sont très efficaces pour augmenter la synthèse des GAGs sulfatés. Cette activité permet d'attribuer à ce composé une activité biologique sur la fermeté de la peau et ainsi d'estomper les signes du vieillissement cutané.

**Exemple 13**

On prépare une crème de soin du visage de type émulsion huile-dans-eau, comprenant (% en poids) :

| | |
|---|---|
| composé de l'exemple 5 | 0,005% |
| stéarate de glycérol | 2% |
| polysorbate 60 (Tween 60 de ICI) | 1% |
| acide stéarique | 1,4% |
| triéthanolamine | 0,7% |
| carbomer | 0,4% |
| fraction liquide du beurre de karité | 12% |
| perhydrosqualène | 12% |
| antioxydant | qs |
| parfum, conservateur | qs |
| eau | qsp 100% |

Une composition similaire est préparée avec les composés des exemples 1 à 4 et 6 à 11.

La composition appliquée sur le visage permet de renforcer la fermeté de la peau et ainsi d'estomper les signes du vieillissement cutané.

**Exemple 14**

On prépare un gel anti-âge pour la peau comprenant (% en poids) :

| | |
|---|---|
| composé de l'exemple 5 | 2% |
| hydroxypropylcellulose (Klucel H de Hercules) | 1% |
| antioxydant | qs |
| parfum, conservateur | qs |
| isopropanol | 40% |
| eau | qsp 100% |

Une composition similaire est préparée avec le composé des exemples 1 à 4 et 6 à 11.

La composition appliquée sur le visage permet de renforcer la fermeté de la peau et ainsi d'estomper les signes du vieillissement cutané.

## Revendications

**1.** Composés de formule (I):

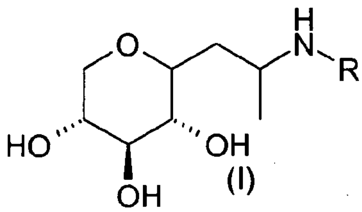

(I)

dans laquelle R représente :

- un radical alkyle linéaire en $C_2$-$C_{18}$, ou
- un radical alkyle cyclique en $C_3$-$C_{18}$, ou
- un radical alkyle ramifié en $C_3$-$C_{10}$, ou
- un radical $-CHR_1-COOR_2$ dans lequel :

$R_1$ représente H ou un radical alkyle linéaire en $C_1$-$C_6$, cyclique en $C_3$-$C_6$ ou ramifié en $C_3$-$C_6$, ledit radical pouvant être éventuellement (i) substitué par au moins un groupement choisi parmi =NH, $-NH_2$, -NHR', $-NR'_2$, =O, -OH, -OR', - SH, -SR', COOR', phényle, phényle substitué par OH ou OR',

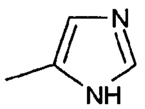

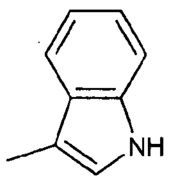

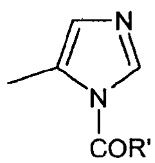

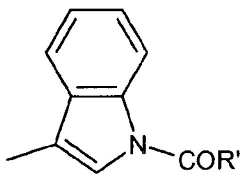

et/ou (ii) interrompu par un groupement -NH-, -N(COR')- ou -S-;
avec R' désignant un radical alkyle linéaire en $C_1$-$C_6$, cyclique en $C_3$-$C_6$, ou ramifié en $C_3$-$C_6$;
$R_2$ représente un radical alkyle linéaire en $C_1$-$C_6$, cyclique en $C_3$-$C_6$ ou ramifié en $C_3$-$C_6$ ;
ainsi que leurs sels, leurs solvates et leurs isomères optiques.

**2.** Composés selon la revendication 1, dans lesquels R représente :

- un radical alkyle linéaire en $C_2$-$C_{16}$, ou
- un radical $-CHR_1-COOR_2$ dans lequel $R_1$ représente un radical alkyle linéaire en $C_1$-$C_4$ ou ramifié en $C_3$-$C_4$, éventuellement substitué par un groupement -OH , COOR', phényle, phényle substitué par OH, R' représentant un radical alkyle linéaire en $C_1$-$C_4$ ou un radical ramifié en $C_3$-$C_4$ ;

$R_2$ représente un radical alkyle linéaire en $C_1$-$C_4$, ou un radical ramifié en $C_3$-$C_4$.

**3.** Composés selon l'une des revendications précédentes, dans lesquels R représente :

- un radical alkyle linéaire en $C_2$-$C_{16}$, ou
- un radical $-CHR_1-COOR_2$ dans lequel $R_1$ un radical alkyle linéaire en $C_1$-$C_2$ ou ramifié en $C_3$-$C_4$, éventuellement substitué par un groupement -OH , un radical -benzyle, un radical hydroxy benzyle, un radical $-CH_2-COOR'$ , R' représentant un radical alkyle linéaire en $C_1$-$C_4$.

**4.** Composés selon l'une des revendications précédentes, choisi parmi les composés suivants :

- ester éthylique de l'acide 2R-[1-méthyl 2- (C- β-D-xylopyranoside) éthyl]-amino 3-hydroxy propanoïque ;
- ester éthylique de l'acide 2R-[1-méthyl 2- (C- β-D-xylopyranoside) éthyl]-amino 3-méthyl butanoïque ;
- ester diéthylique du diacide 2R-[1-méthyl 2- (C- β-D-xylopyranoside) éthyl]-amino 1,4-butanoïque ;
- ester méthylique de l'acide 2R-[1-méthyl 2- (C- β-D-xylopyranoside) éthyl]-amino 3-hydroxy butanoïque ;
- ester éthylique de l'acide 2R-[1-méthyl 2- (C- β-D-xylopyranoside) éthyl]-amino 3-phényl propanoïque ;

- 1-[2-(dodécylamino)-propyl]-C-β-D-xylopyranose) ;
- ester éthylique de l'acide 2R-[1-méthyl 2- (C- β-D-xylopyranoside) éthyl]-amino 3-(4-hydroxy phényl) propanoïque ;
- 1-[2-(hexadécylamino)-propyl]-C-β-D-xylopyranose) ;
- 1-[2-(octylamino)-propyl]-C-β-D-xylopyranose) ;
- 1-[2-(hexylamino)-propyl]-C-β-D-xylopyranose) ;
- 1-[2-(propylamino)-propyl]-C-β-D-xylopyranose) ;

et leurs sels, leurs solvates, et leurs isomères optiques.

5. Composition cosmétique comprenant, dans un milieu physiologiquement acceptable, au moins un composé de formule (I) tel que défini à l'une quelconque des revendications 1 à 4.

6. Composition selon la revendication précédente, dans laquelle le composé de formule (I) est présent, seul ou en mélange, en une quantité comprise entre 0,01 et 10% en poids, de préférence entre 0,1 à 5% en poids, notamment entre 0,5 à 3% en poids, par rapport au poids total de la composition.

7. Composition selon l'une des revendications 5 ou 6, dans laquelle le milieu physiologiquement acceptable comprend au moins un adjuvant cosmétique choisi parmi l'eau; les solvants organiques, notamment les alcools en $C_1$-$C_6$ et les esters d'acide carboxylique en $C_2$-$C_{10}$ ; les huiles hydrocarbonées, les huiles siliconées, les huiles fluorées, les cires, les pigments, les charges, les colorants, les tensioactifs, les émulsionnants, les actifs cosmétiques ou dermatologiques, les filtres UV, les polymères filmogènes, les gélifiants hydrophiles ou lipophiles, les épaississants, les conservateurs, les parfums, les bactéricides, les absorbeurs d'odeur, les antioxydants.

8. Composition selon l'une des revendications 5 à 7, dans laquelle le milieu physiologiquement acceptable comprend au moins un composé choisi parmi: les agents desquamants; les agents hydratants; les agents dépigmentants ou propigmentants; les agents anti-glycation; les inhibiteurs de NO-synthase; les agents stimulant la synthèse de macromolécules dermiques ou épidermiques et/ou empêchant leur dégradation; les agents stimulant la prolifération des fibroblastes et/ou des kératinocytes ou stimulant la différenciation des kératinocytes; les agents myorelaxants et/ou les agents dermo-décontractants; les agents tenseurs; les agents anti-pollution et/ou anti-radicalaire; les agents agissant sur la microcirculation; les agents agissant sur le métabolisme énergétique des cellules; et leurs mélanges.

9. Composition selon l'une des revendications 6 à 8, se présentant sous forme d'une composition anti-âge, notamment de soin destinée à lutter contre les signes extérieurs du vieillissement cutané.

10. Procédé de traitement cosmétique non thérapeutique de la peau, comprenant l'application sur la peau, d'une composition cosmétique telle que définie à l'une des revendications 6 à 9.

11. Procédé selon la revendication précédente dans lequel la composition est appliquée sur la peau mature et/ou ridée.

12. Utilisation non thérapeutique d'une composition cosmétique telle que définie à l'une des revendications 6 à 9, pour améliorer la fermeté de la peau.

13. Utilisation non thérapeutique d'une composition cosmétique telle que définie à l'une des revendications 6 à 9, pour prévenir et/ou traiter cosmétiquement les signes cutanés du vieillissement.

**Patentansprüche**

1. Verbindungen der Formel (I):

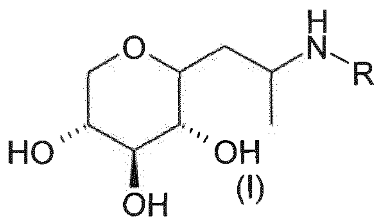

(I)

worin R für

- einen linearen $C_2$-$C_{18}$-Alkylrest oder
- einen cyclischen $C_3$-$C_{18}$-Alkylrest oder
- einen verzweigten $C_3$-$C_{10}$-Alkylrest oder
- einen -$CHR_1$-$COOR_2$-Rest steht, worin:

$R_1$ für H oder einen linearen $C_1$-$C_6$-, cyclischen $C_3$-$C_6$- oder verzweigten $C_3$-$C_6$-Alkylrest steht, wobei der Rest gegebenenfalls (i) durch mindestens eine Gruppe, die aus =NH, -$NH_2$, -NHR', -$NR'_2$, =O, -OH, -OR', -SH, -SR', COOR', Phenyl, durch OH oder OR' substituiertem Phenyl,

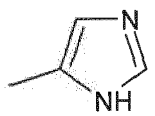
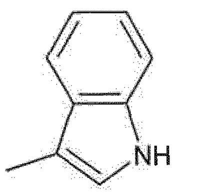
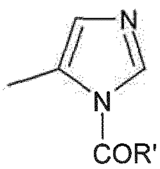
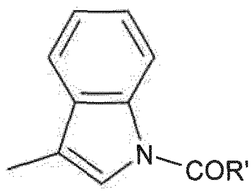

ausgewählt ist, substituiert und/oder (ii) durch eine -NH-, -N(COR')- oder -S-Gruppe unterbrochen sein kann; wobei R' für einen linearen $C_1$-$C_6$-, cyclischen $C_3$-$C_6$- oder verzweigten $C_3$-$C_6$-Alkylrest steht;
$R_2$ für einen linearen $C_1$-$C_6$-, cyclischen $C_3$-$C_6$- oder verzweigten $C_3$-$C_6$-Alkylrest steht;
sowie Salze davon, Solvate davon und optische Isomere davon.

**2.** Verbindungen nach Anspruch 1, worin R für

- einen linearen $C_2$-$C_{16}$-Alkylrest oder
- einen -$CHR_1$-$COOR_2$-Rest steht, worin $R_1$ für einen linearen $C_1$-$C_4$- oder verzweigten $C_3$-$C_4$-Alkylrest steht, der gegebenenfalls durch eine -OH-Gruppe, COOR'-Gruppe, Phenylgruppe, durch OH oder OR' substituierte Phenylgruppe substituiert ist, wobei R' für einen linearen $C_1$-$C_4$-Alkylrest oder einen verzweigten $C_3$-$C_4$-Rest steht;
$R_2$ für einen linearen $C_1$-$C_4$-Alkylrest oder einen verzweigten $C_3$-$C_4$-Rest steht.

**3.** Verbindungen nach einem der vorhergehenden Ansprüche, worin R für

- einen linearen $C_2$-$C_{16}$-Alkylrest oder
- einen -$CHR_1$-$COOR_2$-Rest steht, worin $R_1$ für einen linearen $C_1$-$C_2$- oder verzweigten $C_3$-$C_4$-Alkylrest, der gegebenenfalls durch eine -OH-Gruppe substituiert ist, einen Benzylrest, einen Hydroxybenzylrest oder einen -$CH_2$-COOR'-Rest steht, wobei R' für einen linearen $C_1$-$C_4$-Alkylrest steht.

**4.** Verbindungen nach einem der vorhergehenden Ansprüche, ausgewählt aus den folgenden Verbindungen:

- 2R-[1-Methyl-2-(C-β-D-xylopyranosid)ethyl]amino-3-hydroxypropansäureethylester;
- 2R-[1-Methyl-2-(C-β-D-xylopyranosid)ethyl]amino-3-methylbutansäureethylester;
- 2R-[1-Methyl-2-(C-β-D-xylopyranosid)ethyl]amino-1,4-butandisäurediethylester;
- 2R-[1-Methyl-2-(C-β-D-xylopyranosid)ethyl]amino-3-hydroxybutansäuremethylester;
- 2R-[1-Methyl-2-(C-β-D-xylopyranosid)ethyl]amino-3-phenylpropansäureethylester;
- 1-[2-(Dodecylamino)propyl]-C-β-D-xylopyranose;
- 2R-[1-Methyl-2-(C-β-D-xylopyranosid)ethyl]amino-3-(4-hydroxyphenyl)propansäureethylester;

- 1-[2-(Hexadecylamino)propyl]-C-β-D-xylopyranose;
- 1-[2-(Octylamino)propyl]-C-β-D-xylopyranose;
- 1-[2-(Hexylamino)propyl]-C-β-D-xylopyranose;
- 1-[2-(Propylamino)propyl]-C-β-D-xylopyranose; und Salzen davon, Solvaten davon und optischen Isomeren davon.

**5.** Kosmetische Zusammensetzung, die in einem physiologisch unbedenklichen Medium mindestens eine Verbindung der Formel (I) gemäß einem der Ansprüche 1 bis 4 umfasst.

**6.** Zusammensetzung nach dem vorhergehenden Anspruch, in der die Verbindung der Formel (I) allein oder als Gemisch in einer Menge zwischen 0,01 und 10 Gew.-%, vorzugsweise zwischen 0,1 und 5 Gew.-%, insbesondere zwischen 0,5 und 3 Gew.-%, bezogen auf das Gesamtgewicht der Zusammensetzung, vorliegt.

**7.** Zusammensetzung nach Anspruch 5 oder 6, in der das physiologisch unbedenkliche Medium mindestens einen kosmetischen Hilfsstoff, der aus Wasser; organischen Lösungsmitteln, insbesondere $C_1$-$C_6$-Alkoholen und $C_2$-$C_{10}$-Carbonsäureestern; Kohlenwasserstoffölen, Silikonölen, fluorierten Ölen, Wachsen, Pigmenten, Füllstoffen, Farbmitteln, Tensiden, Emulgatoren, kosmetischen oder dermatologischen Wirkstoffen, UV-Schutzmitteln, filmbildenden Polymeren, hydrophilen oder lipophilen Geliermitteln, Verdickungsmitteln, Konservierungsmitteln, Duftstoffen, Bakteriziden, Geruchsabsorptionsmitteln und Antioxidantien ausgewählt ist, umfasst.

**8.** Zusammensetzung nach einem der Ansprüche 5 bis 7, wobei das physiologisch unbedenkliche Medium mindestens eine Verbindung, die aus Entschuppungsmitteln; Feuchtigkeitsmitteln; depigmentierenden oder propigmentierenden Mitteln; Antiglykierungsmitteln; NO-Synthase-Inhibitoren; Mitteln zur Stimulierung der Synthese von dermalen oder epidermalen Makromolekülen und/oder zur Verhinderung ihrer Degradation; Mitteln zur Stimulierung der Fibroblasten- und/oder Keratinozytenproliferation oder zur Stimulierung der Keratinozytendifferenzierung; Muskelrelaxantien und/oder Hautdekontraktionsmitteln; Straffungsmitteln; Mitteln gegen Schadstoffe und/oder Radikale; Mitteln, die auf die Mikrozirkulation wirken; Mitteln, die auf den Energiestoffwechsel von Zellen wirken; und Mischungen davon ausgewählt ist, umfasst.

**9.** Zusammensetzung nach einem der Ansprüche 6 bis 8, die in Form einer Alterungsschutzzusammensetzung, insbesondere einer Pflegezusammensetzung zur Bekämpfung der äußeren Zeichen der Hautalterung, vorliegt.

**10.** Verfahren zur nichttherapeutischen kosmetischen Behandlung der Haut, bei dem man auf die Haut eine kosmetische Zusammensetzung gemäß einem der Ansprüche 6 bis 9 aufbringt.

**11.** Verfahren nach dem vorhergehenden Anspruch, bei dem man die Zusammensetzung auf reife und/oder faltige Haut aufbringt.

**12.** Nichttherapeutische Verwendung einer kosmetischen Zusammensetzung gemäß einem der Ansprüche 6 bis 9 zur Verbesserung der Straffheit der Haut.

**13.** Nichttherapeutische Verwendung einer kosmetischen Zusammensetzung gemäß einem der Ansprüche 6 bis 9 zur Prävention und/oder kosmetischen Behandlung der Zeichen der Hautalterung.

## Claims

**1.** Compounds of formula (I):

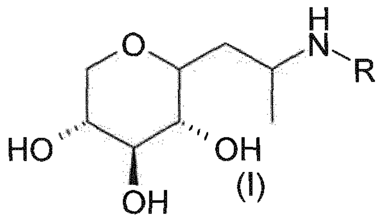

(I)

in which R represents:

- a linear $C_2$-$C_{18}$ alkyl radical, or
- a cyclic $C_3$-$C_{18}$ alkyl radical, or
- a branched $C_3$-$C_{10}$ alkyl radical, or
- a $-CHR_1-COOR_2$ radical in which:

$R_1$ represents H or a linear $C_1$-$C_6$, cyclic $C_3$-$C_6$ or branched $C_3$-$C_6$ alkyl radical, it being possible for said radical to be optionally (i) substituted with at least one group chosen from =NH, $-NH_2$, -NHR', $-NR'_2$, =O, -OH, -OR', -SH, -SR', COOR', phenyl, phenyl substituted with OH or OR',

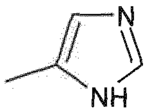
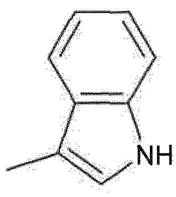
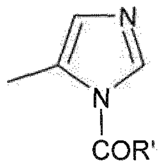
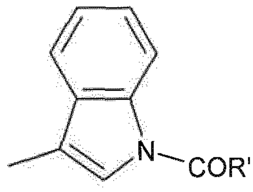

and/or (ii) interrupted with an -NH-, -N(COR')- or -S- group;
with R' denoting a linear $C_1$-$C_6$, cyclic $C_3$-$C_6$ or branched $C_3$-$C_6$ alkyl radical;
$R_2$ represents a linear $C_1$-$C_6$, cyclic $C_3$-$C_6$ or branched $C_3$-$C_6$ alkyl radical; and also the salts thereof, the solvates thereof and the optical isomers thereof.

**2.** Compounds according to Claim 1, in which R represents:

- a linear $C_2$-$C_{16}$ alkyl radical, or
- a $-CHR_1-COOR_2$ radical in which $R_1$ represents a linear $C_1$-$C_4$ or branched $C_3$-$C_4$ alkyl radical, optionally substituted with an -OH, COOR' or phenyl group, or a phenyl group substituted with OH, R' representing a linear $C_1$-$C_4$ alkyl radical or a branched $C_3$-$C_4$ radical;
$R_2$ represents a linear $C_1$-$C_4$ alkyl radical, or a branched $C_3$-$C_4$ radical.

**3.** Compounds according to either of the preceding claims, in which R represents:

- a linear $C_2$-$C_{16}$ alkyl radical, or
- a $-CHR_1-COOR_2$ radical in which $R_1$ represents a linear $C_1$-$C_2$ or branched $C_3$-$C_4$ alkyl radical, optionally substituted with an -OH group, a -benzyl radical, a hydroxybenzyl radical, or a $-CH_2-COOR'$ radical, R' representing a linear $C_1$-$C_4$ alkyl radical.

**4.** Compounds according to one of the preceding claims, chosen from the following compounds:

- ethyl ester of 2R-[1-methyl-2-(C-β-D-xylopyranoside)ethyl]amino-3-hydroxypropanoic acid;
- ethyl ester of 2R-[1-methyl-2-(C-β-D-xylopyranoside)ethyl]amino-3-methylbutanoic acid;
- diethyl ester of 2R-[1-methyl-2-(C-β-D-xylopyranoside)ethyl]amino-1,4-butanedioic acid;
- methyl ester of 2R-[1-methyl-2-(C-β-D-xylopyranoside)ethyl]amino-3-hydroxy butanoic acid;
- ethyl ester of 2R-[1-methyl-2-(C-β-D-xylopyranoside)ethyl]amino-3-phenyl propanoic acid;
- 1-[2-(dodecylamino)propyl]-C-β-D-xylopyranose;
- ethyl ester of 2R-[1-methyl-2-(C-β-D-xylopyranoside)ethyl]amino-3-(4-hydroxyphenyl)propanoic acid;
- 1-[2-(hexadecylamino)propyl]-C-β-D-xylopyranose;
- 1-[2-(octylamino)propyl]-C-β-D-xylopyranose;
- 1-[2-(hexylamino)propyl]-C-β-D-xylopyranose;
- 1-[2-(propylamino)propyl]-C-β-D-xylopyranose;

and the salts thereof, the solvates thereof and the optical isomers thereof.

**5.** Cosmetic composition comprising at least one compound of formula (I) as defined in any one of Claims 1 to 4, in a physiologically acceptable medium.

**6.** Composition according to the preceding claim, in which the compound of formula (I) is present, alone or as a mixture,

in an amount of between 0.01% and 10% by weight, preferably between 0.1% and 5% by weight, in particular between 0.5 and 3% by weight, relative to the total weight of the composition.

7. Composition according to either of Claims 5 and 6, in which the physiologically acceptable medium comprises at least one cosmetic adjuvant chosen from water; organic solvents, in particular $C_1$-$C_6$ alcohols and $C_2$-$C_{10}$ carboxylic acid esters; hydrocarbon-based oils, silicone oils, fluoro oils, waxes, pigments, fillers, dyes, surfactants, emulsifiers, cosmetic or dermatological active agents, UV-screening agents, film-forming polymers, hydrophilic or lipophilic gelling agents, thickeners, preservatives, fragrances, bactericides, odor absorbers and antioxidants.

8. Composition according to one of Claims 5 to 7, in which the physiologically acceptable medium comprises at least one compound chosen from: desquamating agents; moisturizing agents; depigmenting or propigmenting agents; anti-glycation agents; NO-synthase inhibitors; agents which stimulate the synthesis of dermal or epidermal macromolecules and/or prevent degradation thereof; agents which stimulate fibroblast and/or keratinocyte proliferation or stimulate keratinocyte differentiation; muscle relaxants and/or dermo-decontracting agents; tensioning agents; anti-pollution agents and/or free-radical scavengers; agents which act on the microcirculation; agents which act on the energy metabolism of cells; and mixtures thereof.

9. Composition according to one of Claims 6 to 8, which is in the form of an anti-aging, in particular care, composition intended for combating the external signs of aging of the skin.

10. Method for nontherapeutic cosmetic treatment of the skin, comprising the application, to the skin, of a cosmetic composition as defined in one of Claims 6 to 9.

11. Method according to the preceding claim, in which the composition is applied to mature and/or wrinkled skin.

12. Nontherapeutic use of a cosmetic composition as defined in one of Claims 6 to 9, for improving the firmness of the skin.

13. Nontherapeutic use of a cosmetic composition as defined in one of Claims 6 to 9, for preventing and/or cosmetically treating the signs of aging on the skin.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- WO 02051828 A